# EUROPÄISCHE PATENTANMELDUNG

(11) **EP 1 332 782 A1**
(43) Veröffentlichungstag der Anmeldung: **06.08.2003**
(21) Anmeldenummer: 03000488.1
(22) Anmeldetag: 11.01.2003
(51) Int. Cl.: B01D 53/04, A61M 16/10

(54) **Verfahren und Vorrichtung zur Bereitstellung von Atemgas**

(30) Priorität: 05.02.2002 DE 10204467
(71) Anmelder: Gottlieb Weinmann Geräte für Medizin und Arbeitsschutz GmbH & Co.KG, 22525 Hamburg (DE)
(72) Erfinder: Feldhahn, Karl-Andreas, Dr., 22761 Hamburg (DE); Palm, Ulrich, 22848 Norderstedt (DE)
(74) Vertreter: Klickow, Hans-Henning

(57) **Zusammenfassung**

Das Verfahren und die Vorrichtung dienen zur Bereitstellung von Atemgas. Das Atemgas wird von einem Sauerstoffkonzentrator (7) bereitgestellt und durch eine Befülleinrichtung (1) für Druckgasflaschen (5) durch eine Sauerstoffbrille (9) einem Patienten zugeführt. Der Druckgasflasche (5) wird mindestens zeitweilig mindestens ein Teil des vom Sauerstoffkonzentrator (7) bereitgestellten Atemgases zugeführt. Im Bereich der Befülleinrichtung (1) wird das zur Befüllung der Druckgasflasche (5) vorgesehene Atemgas von einem Verdichter (3) unabhängig von einer Funktion des Sauerstoffkonzentrators (7) komprimiert. Der Befüllvorgang der Druckgasflasche (5) wird in Abhängigkeit von mindestens einem Meßwert (11) gesteuert, der einem aktuellen Atemgasbedarf im Bereich der Sauerstoffbrille (9) entspricht. Der Meßwert (11) wird entweder sensorisch erfaßt oder von einer Steuerungsvorgabe übernommen.

## Beschreibung

Die Erfindung betrifft ein Verfahren zur Bereitstellung von Atemgas, bei dem einer Sauerstoffbrille für einen Patienten von einem Sauerstoffkonzentrator bereitgestelltes Atemgas zugeführt wird.

Die Erfindung betrifft darüber hinaus eine Vorrichtung zur Bereitstellung von Atemgas, die einen Sauerstoffkonzentrator sowie eine an den Sauerstoffkonzentrator anschließbare Sauerstoffbrille aufweist.

Sauerstoffkonzentratoren werden typischerweise verwendet, um stationär Patienten mit entsprechenden Krankheitssymptomen mit Atemgas zu versorgen, das eine starke Anreicherung an Sauerstoff aufweist. Typischerweise werden die Sauerstoffkonzentratoren im Bereich einer Wohnung des Patienten bzw. in Arztpraxen oder Krankenhäusern installiert. Für eine mobile Versorgung der Patienten werden Druckgasflaschen verwendet, die der Patient mitführen kann und mit denen der Patient in die Lage versetzt wird, zumindest bei einem Auftreten von akuten Symptomen unmittelbar selbst eine Versorgung mit einer erhöhten Sauerstoffkonzentration durchführen zu können.

Der Benutzer von derartigen Druckgasflaschen besitzt entweder selbst eine eigene Befülleinrichtung für diese Druckgasflaschen, die an einen hierfür vorgesehenen speziellen Sauerstoffkonzentrator angeschlossen werden kann, oder tauscht bei entsprechenden Anbietern seine geleerte Druckgasflasche gegen eine gefüllte Druckgasflasche ein.

Die bekannten Verfahren und Vorrichtungen ermöglichen es nicht, eine Befüllung der Druckgasflaschen mit Sauerstoffkonzentratoren unterschiedlicher Bauart durchzuführen, darüber hinaus ist es auch noch nicht in ausreichender Weise möglich, parallel sowohl eine Atemgasversorgung eines Patienten als auch eine Befüllung von Druckgasflaschen durchführen zu können.

Aufgabe der vorliegenden Erfindung ist es daher, ein Verfahren der einleitend genannten Art derart zu verbessern, daß eine erhöhte Nutzungsflexibilität bereitgestellt wird.

Diese Aufgabe wird erfindungsgemäß dadurch gelöst, daß einer Druckgasflasche zur Aufnahme von mit Sauerstoff angereichertem Atemgas mindestens zeitweilig mindestens ein Teil des vom Sauerstoffkonzentrator bereitgestellten Atemgases zugeführt wird und daß das vom Sauerstoffkonzentrator erzeugte Atemgas durch eine Befülleinrichtung hindurch geleitet wird, in deren Bereich das Atemgas zur Befüllung der Druckgasflasche von einem vom Sauerstoffkonzentrator unabhängigen Verdichter komprimiert wird, sowie daß der Befüllvorgang der Druckgasflasche in Abhängigkeit von mindestens einem Meßwert gesteuert wird, der in Abhängigkeit von einem aktuellen Atemgasbedarf im Bereich der Sauerstoffbrille veränderlich ist.

Weitere Aufgabe der Erfindung ist es, eine Vorrichtung der einleitend genannten Art derart zu konstruieren, daß eine erhöhe Mobilität für den Patienten bereitgestellt wird.

Diese Aufgabe wird erfindungsgemäß dadurch gelöst, daß im Bereich einer Verbindungsleitung zwischen dem Sauerstoffkonzentrator und der Sauerstoffbrille eine Befülleinrichtung für Druckgasflaschen angeordnet ist, daß die Befülleinrichtung einen vom Sauerstoffkonzentrator unabhängigen Verdichter zur Kompression von Vorratsgas für die Druckgasflasche sowie eine Steuereinrichtung für eine Verteilung des vom Sauerstoffkonzentrator erzeugten Atemgases aufweist und daß an die Steuereinrichtung mindestens ein Sensor zur Erfassung eines aktuellen Verbrauches an Atemgas im Bereich der Sauerstoffbrille angeschlossen ist.

Durch die Anordnung einer vom Sauerstoffkonzentrator unabhängigen Befülleinrichtung zwischen dem Sauerstoffkonzentrator und der Sauerstoffbrille ist es möglich, konstruktiv unterschiedliche Sauerstoffkonzentratoren und insbesondere auch Sauerstoffkonzentratoren unterschiedlicher Hersteller zur Befüllung der Druckgasflaschen einzusetzen. Hierdurch wird es einem Patienten ermöglicht, seine individuelle Druckgasflasche nicht nur mit Hilfe seines eigenen Sauerstoffkonzentrators wieder zu befüllen, der in der Regel in der Wohnung des Patienten angeordnet ist, sondern der Patient kann eine Befüllung auch unter Verwendung anderer Sauerstoffkonzentratoren vorzunehmen. Hierdurch wird zum einen die Mobilität des Patienten erheblich erhöht, darüber hinaus können auch bei stationären Anwendungen durch die Möglichkeit zu einem Anschluß unterschiedlicher Sauerstoffkonzentratoren verbesserte Einsatzbedingungen geschaffen werden und der Patient ist nicht an einen bestimmten Hersteller von Sauerstoffkonzentratoren gebunden.

Das Verfahren und die Vorrichtung ermöglichen es darüber hinaus, die Befüllung der Druckgasflasche parallel zu einer Beatmung des Patienten durchzuführen und hierdurch bereits nach möglichst kurzer Zeit wieder eine befüllte Druckgasflasche bereitzustellen. Es ist somit nicht mehr erforderlich, den Sauerstoffkonzentrator entweder nur zur Beatmung des Patienten oder nur zur Befüllung der Druckgasflasche einzusetzen, sondern bereits während der Beatmung des Patienten kann aktuell für die Beatmung nicht benötigtes mit Sauerstoff angereichertes Atemgas unter Verwendung des Verdichters in die Druckgasflasche eingeleitet werden.

Zur Unterstützung einer langen Betriebsfähigkeit wird vorgeschlagen, daß eine mehrstufige Verdichtung des Atemgases durchgeführt wird.

Insbesondere erweist es sich als zweckmäßig, daß eine zweistufige Verdichtung des Atemgases durchgeführt wird.

Zur Sicherstellung einer ausreichenden Sauerstoffversorgung des Patienten wird vorgeschlagen, daß eine Messung der Sauerstoffkonzentration des Atemgases durchgeführt wird.

Ebenfalls trägt es zur Sicherstellung einer ordnungsgemäßen Atemgasversorgung bei, daß eine Messung des Volumenflusses an Atemgas durchgeführt wird.

Eine einfache Handhabung der Befülleinrichtung wird dadurch unterstützt, daß ein Volumenfluß an Atemgas, der in Richtung auf die Sauerstoffbrille geleitet wird, fest eingestellt wird und daß ein vom Sauerstoffkonzentrator bereitgestellter Volumenfluß, der diesen eingestellten Wert übersteigt, dem Verdichter zugeführt wird.

Eine universelle Anwendbarkeit wird dadurch unterstützt, daß von der Befülleinrichtung eine Verteilung von Atemgas durchgeführt wird, das von konstruktiv unterschiedlichen Sauerstoffkonzentratoren bereitgestellt wird.

Zur Verbesserung der physiologischen Eigenschaften des Atemgases wird vorgeschlagen, daß eine Befeuchtung des in Richtung auf die Sauerstoffbrille abgeleiteten Atemgases durchgeführt wird.

Eine visuelle Überwachung der Vorrichtung wird dadurch unterstützt, daß im Bereich der Befülleinrichtung mindestens ein Betriebsparameter angezeigt wird.

Zur Nutzung von typischerweise im Bereich von Sauerstoffkonzentratoren vorhandenen Kompressoren wird vorgeschlagen, daß eine erste Stufe des Verdichters im Bereich des Sauerstoffkonzentrators und eine zweite Stufe des Verdichters im Bereich der Befülleinrichtung angeordnet ist.

Eine weitere Vergrößerung des Anwendungsgebietes läßt sich dadurch erreichen, daß die Befülleinrichtung einen Adapter zum Anschluß relativ zueinander konstruktiv unterschiedlicher Sauerstoffkonzentratoren aufweist.

In den Zeichnungen sind Ausführungsbeispiele der Erfindung schematisch dargestellt. Es zeigen:
Fig. 1 Eine schematische Darstellung einer Befülleinrichtung, an die ein Sauerstoffkonzentrator, ein Befeuchter mit weiterer Verbindung zu einer Sauerstoffbrille sowie eine Druckgasflasche angeschlossen sind,
Fig. 2 eine perspektivische Darstellung eines Sauerstoffkonzentrators mit abgenommenem Gehäuse,
Fig. 3 eine perspektivische Darstellung des Konzentrators gemäß Fig. 2 bei einer Blickrichtung schräg aus der Zeichenebene in Fig. 2 heraus,
Fig. 4 eine perspektivische Darstellung einer Druckgasflasche mit Druckarmaturen sowie angeschlossener Verbrauchssteuerung und Sauerstoffbrille und
Fig. 5 eine vergrößerte Darstellung der Verbrauchssteuerung mit über einen Verbindungsschlauch angeschlossener Sauerstoffbrille sowie teilweise dargestelltem Verbindungsschlauch zum Anschluß an die Druckgasflasche.

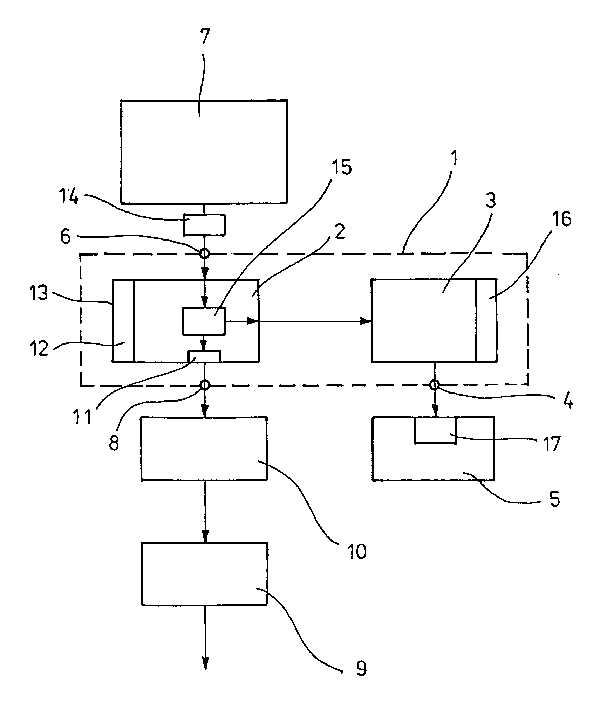

Fig. 1 zeigt eine Befülleinrichtung (1), die mit einer Steuereinrichtung (2) sowie einem Verdichter (3) versehen ist. Der Verdichter (3) ist über einen Befüllausgang (4) mit einer Druckgasflasche (5) verbindbar. Die Befülleinrichtung (1) ist über einen Atemgaseingang (6) an einen Sauerstoffkonzentrator (7) angeschlossen und über einen Atemgasausgang (8) kann eine Verbindung zu einer Sauerstoffbrille (9) realisiert werden. Der Anschluß an die Sauerstoffbrille (9) kann unter Zwischenschaltung eines Befeuchters (10) erfolgen.

An die Steuereinrichtung (2) ist ein Sensor (11) für die Erfassung eines aktuellen Verbrauches an Atemgas im Bereich der Sauerstoffbrille (9) angeschlossen. Der Sensor (11) kann beispielsweise als ein Sensor zur Erfassung einer Sauerstoffkonzentration und / oder zur Erfassung eines Volumenflusses an Atemgas ausgebildet sein. Darüber hinaus ist es auch möglich, die Steuereinrichtung (2) mit einem Einstellelement (12) zur Vorgabe eines Volumenflusses von Atemgas in Richtung auf die Sauerstoffbrille (9) auszustatten. Bei Verwendung eines derartigen Einstellelementes (12) kann der Sensor (11) als Erfassungseinrichtung für eine aktuelle Einstellung des Einstellelementes (12) realisiert sein. Darüber hinaus ist es auch möglich, auf eine sensorische Erfassung zu verzichten und lediglich Einstellvorgaben als Sollwerte auszuwerten. Der Begriff des Sensors (11) umfaßt somit auch die Übernahme eines vorgegebenen Wertes ohne Durchführung einer Messung

Beim Ausführungsbeispiel gemäß Fig. 1 ist die Befülleinrichtung (1) mit einer Anzeige (13) für mindestens einen Betriebparameter versehen. Darüber hinaus ist zwischen dem Atemgaseingang (6) und dem Sauerstoffkonzentrator (7) ein Adapter (14) angeordnet, um einen Anschluß von Sauerstoffkonzentratoren (7) mit unterschiedlich gestalteten Abgabeelementen zu unterstützen.

Die Steuereinrichtung (2) ist mit einem Verteiler (15) versehen, der in Abhängigkeit von jeweiligen Steuerungsvorgaben eine Weiterleitung des Atemgases in Richtung auf die Sauerstoffbrille (9) und / oder in Richtung auf den Verdichter (3) vorgibt. Der Verdichter (3) ist mit einem Antrieb (16) ausgestattet und im Bereich der Druckgasflasche (5) ist ein Flaschenventil (17) angeordnet, um bei einer Entleerung der Druckgasflasche (5) einen jeweiligen Öffnungs- oder Schließzustand vorgeben zu können. Das Flaschenventil (17) kann bei einer Benutzung der Druckgasflasche (5) durch einen Patienten zusätzlich mit einem Druckminderer gekoppelt werden, um einen jeweiligen Verbrauchsdruck vorzugeben.

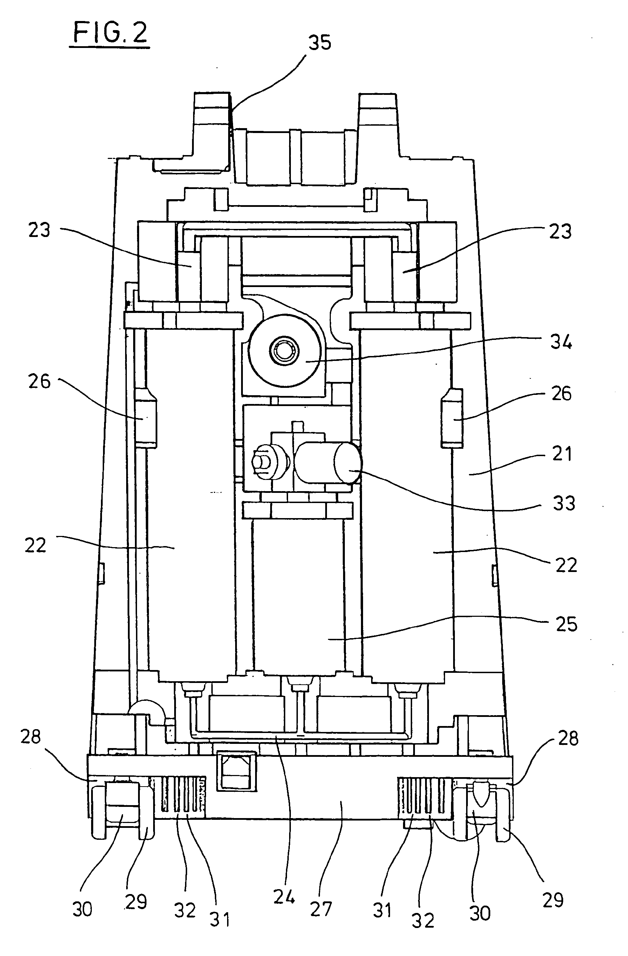

Fig. 2 zeigt in einer rückwärtigen Ansicht den Sauerstoffkonzentrator (7) bei abgenommenem Gerätegehäuse. Ein Träger (21) des Sauerstoffkonzentrators (7) haltert bezüglich ihrer Längsachsen im Wesentlichen vertikal angeordnete Filterelemente (22), die bei einer Gasdurchströmung Stickstoff zurückhalten. Beispielsweise ist es möglich, die Filterelemente (22) mit einer im Wesentlichen zylindrischen Gehäuseform zu versehen und die zu trennende Luft über Ventile (23) in einem Kopfbereich der Filterelemente (22) zuzuführen. Abströmende und mit Sauerstoff angereicherte Luft wird über Verbindungsleitungen (24) in den Bereich eines Sammelbehälters (25) geleitet, der ebenfalls zylindrisch konstruiert sein kann.

Die Filterelemente (22) können derart ausgebildet sein, daß in ihrem Inneren ein granulatartiges Material angeordnet wird, an das sich Stickstoff bei einer Durchströmung anlagert und das Sauerstoff passieren läßt. Geeignet sind vor allem Materialien auf Basis von Zeolithen.

Der Träger (21) weist zur Halterung der Filterelemente (22) und des Sammelbehälters (25) muldenförmige Vertiefungen auf. Ein Festklemmen der Filterelemente (22) in den muldenförmigen Vertiefungen kann über Klemmzungen (26) erfolgen. Der Träger (21) erstreckt sich mit einer Grundfläche im Wesentlichen vertikal, so daß die gehalterten Bauelemente in horizontaler Richtung eingesetzt werden können.

In einem Bodenbereich mündet der Träger (21) in eine Grundplatte (27) ein, die vorzugsweise einteilig mit dem Träger (21) ausgebildet ist. Die Grundplatte (27) weist in Vertiefungen (28) angeordnete Räder (29) auf, die von schwenkbaren Radgestellen (30) gehaltert sind.

Durch die Grundplatte (27) hindurch erstrecken sich Lüftungskanäle (31) für Kühlluft, die im Bereich ihrer Überleitung in eine Umgebung mit einer labyrinthartigen Ausströmungsstruktur zur Geräuschminderung versehen sind.

Oberhalb des Sammelbehälters (25) ist ein Druckminderer (33) angeordnet, des weiteren ist vom Träger (21) ein Ventilator (34) zur Erzeugung einer erforderlichen Kühlluftströmung durch die Lüftungskanäle (31) hindurch gehaltert.

Die Ventile (23), die vorzugsweise als Drei-Zwei-Wegeventile ausgebildet sind, sind über Verrohrungen eingangsseitig an einen Feinfilter sowie einen vorgeschalteten Grobfilter zur Zurückhaltung von Luftverunreinigungen angeschlossen. Als Ventile (23) können beispielsweise ebenfalls Fünf-Drei-Wegeventile bzw. Ventile mit Mittelstellung verwendet werden. Ausgangsseitig weisen die Ventile (23) eine Verbindung zu Schalldämpfern auf.

Über eine geeignete Ansteuerung der Ventile (23) ist es möglich, eine abwechselnde Aktivierung der beiden Filterelemente (22) vorzunehmen. Nach einer Deaktivierung eines der Filterelemente (22) erfolgt in diesem Filterelement (22) ein partielles Rückströmen von Luft, so daß der im Filterelement (22) zurückgehaltene Stickstoff wieder ausgetragen und an die Umgebung abgegeben wird. Durch dieses Regenerationsprinzip wird nach Durchlaufen jedes alternierenden Arbeitszyklusses wieder der Ausgangszustand bereitgestellt, so daß Wartungsarbeiten weitgehend entbehrlich sind. Insbesondere ist es nicht erforderlich, die Filterelemente (22) aufgrund zunehmender Stickstoffansammlungen auszuwechseln.

In lotrechter Richtung oben sind in den Träger (21) Haltebügel (35) eingebettet, die zur Befestigung eines Handgriffes dienen. Der Handgriff wird nach einem Aufsetzen des nicht dargestellten Gerätegehäuses auf den Träger (21) montiert und verbindet nach einer entsprechenden Befestigung den Träger (21) mit dem äußeren Gerätegehäuse.

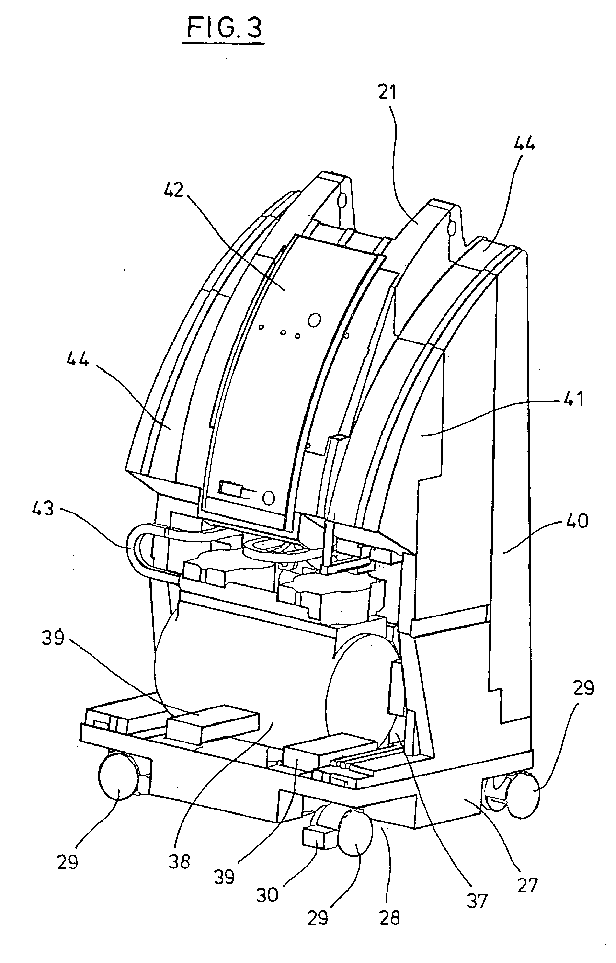

Aus der perspektivischen Darstellung in Fig. 3 ist erkennbar, daß innerhalb eines abgegrenzten Kompressorraumes (37) ein Kompressor (38) angeordnet ist. Der Kompressorraum erstreckt sich oberhalb der Grundplatte (27). Der Kompressor (38) steht ohne separate Befestigung auf Lagerblöcken (39) und wird ebenfalls durch Formschluß fixiert.

Aus der Darstellung ist ebenfalls erkennbar, daß im rückwärtigen Teil der Träger (21) mit den gehalterten Bauelementen von einem Rückenteil (40) abgedeckt ist. Oberhalb des Kompressors (38) erstreckt sich eine vom Träger (21) abnehmbare Abdeckschale (41), die ein Bedienfeld (42) trägt.

Durch die Anordnung des Kompressors (38) in einem separaten Kompressorraum (37) und die Lagerung auf den Lagerblöcken (39) wird eine sehr gute Schall- und Vibrationsentkopplung bereitgestellt. Kühlelemente (43) werden von Kühlluft umströmt, die durch die Lüftungskanäle (31) hindurch in die Umgebung entweichen kann. Die von den Filterelementen (22) bereitgestellte und mit Sauerstoff angereicherte Luft wird in separaten Luftleitungen getrennt von der Kühlluft geführt.

Der Träger (21), die Abdeckschale (41), das Rückenteil (40) sowie ein nicht dargestelltes Abdeckelement für den Kompressorraum (27) werden von Gurten zusammengehalten, die mit Schnellverschlüssen versehen sind. Es kann hierdurch eine sehr schnelle Montage und Demontage unterstützt werden.

Aus der Darstellung in Fig. 3 ist ebenfalls erkennbar, daß der Träger (21), das Rückenteil (40), die Abdeckschale (41) sowie die Verschlußschale für den Kompressorraum (37) eine stufenförmige Kontur aufweisen, um nach einem Zusammenfügen eine Selbstausrichtung durchzuführen. Es wird hierdurch eine exakt definierte und reproduzierbare Einbausituation bereitgestellt.

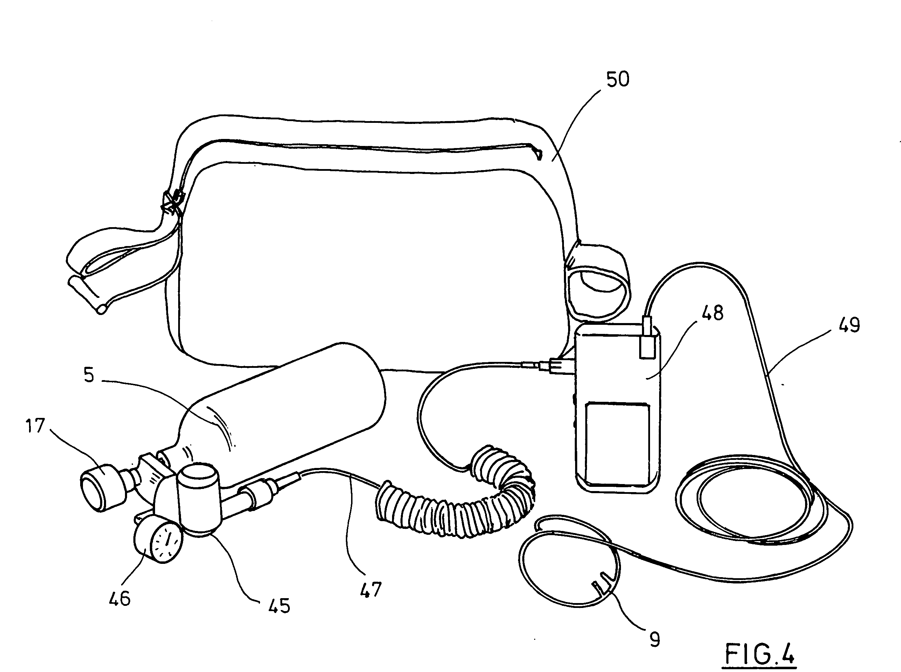

Fig. 4 zeigt die Druckgasflasche (5) mit Flaschenventil (17) sowie angeschlossener Sauerstoffbrille (9). An das Flaschenventil (17) ist ein Druckminderer (45) mit Manometer (46) zur Anzeige eines jeweiligen Verbrauchsdrucks angeschlossen. Der Druckminderer ist über einen Anschlußschlauch (47) mit der Verbrauchssteuerung (48) verbunden. Der Anschlußschlauch (47) kann als ein Spiralschlauch ausgebildet sein, um sowohl eine kompakte Gestaltung als auch unterschiedliche Verbindungslängen bereit zu stellen. An die Verbrauchssteuerung (48) ist die Sauerstoffbrille (9) über eine Versorgungsleitung (49) angeschlossen.

Der Druckminderer (45) gewährleistet, daß der Ausgangsdruck im Wesentlichen unabhängig vom Füllzustand der Druckgasflasche (5) konstant gehalten wird. Zur Vorgabe eines Volumenflusses an abgegebenem Sauerstoff kann eine Lochblende eingesetzt werden. Die Verbrauchssteuerung (48) erfaßt über einen geeigneten Sensor einen vom Patienten erzeugten Einatemimpuls und steuert in Abhängigkeit von diesem erfaßten Meßwert über ein Ventil, das im Bereich der Verbrauchssteuerung (48) angeordnet ist, die Sauerstoffabgabe. Eine typische Öffnungszeit dieses Ventils beträgt etwa 40 Millisekunden je Einatmungsphase.

Zur Unterstützung einer Mobilität des Patienten kann die gesamte in Fig. 4 dargestellte Einrichtung in einer Transporttasche (50) untergebracht werden. Die Transporttasche (50) ermöglicht eine hohe Mobilität des Patienten und schützt die Einrichtung in einem unbenutzten Zustand gegenüber von Beschädigungen.

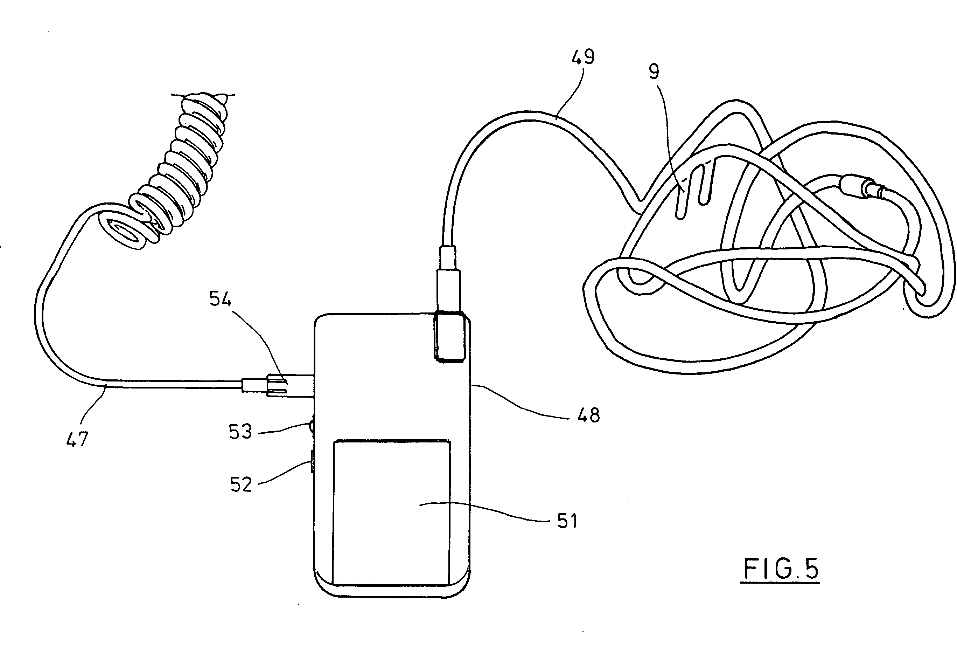

Fig. 5 veranschaulicht in etwas stärkerer Detailliertheit den Aufbau der Verbrauchssteuerung (48). Die Verbrauchssteuerung (48) ist mit einer Klappe (51) versehen, um einen Zugang zu einem Aufnahmefach für Akkumulatoren oder Batterien zu ermöglichen. Hierdurch wird eine netzunabhängige mobile Betriebsweise unterstützt. Ein Netzbetrieb oder eine Aufladung eingesetzter Akkumulatoren kann unter Verwendung einer Ladebuchse (52) durchgeführt werden. Eine Betriebsbereitschaft wird über eine Leuchtdiode (53) signalisiert. Zur Erleichterung einer Ankopplung des Anschlußschlauches (47) ist im Bereich der Verbrauchssteuerung (48) eine Schnellkupplung (54) angeordnet.

Zum Anschluß einer Sauerstoffbrille (9) an die Befülleinrichtung (1) bei gleichzeitiger Befüllung der Druckgasflasche (5) wird typischerweise zunächst der Druckminderer (45) vom Flaschenventil (17) abgeschraubt und anschließend die Druckgasflasche (5) an die Befülleinrichtung (1) angekoppelt. Der Druckminderer (45) mit Versorgungsleitung (49) wird bei einer Befüllung der Druckgasflasche (5) typischerweise nicht für eine Versorgung des Patienten verwendet, sondern der Patient verwendet hier in der Regel einen längeren Verbindungsschlauch zu einer anderen Sauerstoffbrille (9), damit ein relativ großer Bewegungsbereich für den Patienten in dessen Wohnung oder in einem anderen Aufenthältsraum bereitgestellt wird.

## Patentansprüche

1. Verfahren zur Bereitstellung von Atemgas, bei dem einer Sauerstoffbrille für einen Patienten von einem Sauerstoffkonzentrator bereitgestelltes Atemgas zugeführt wird, **dadurch gekennzeichnet, daß** einer Druckgasflasche (5) zur Aufnahme von mit Sauerstoff angereichertem Atemgas mindestens zeitweilig mindestens ein Teil des vom Sauerstoffkonzentrator (7) bereitgestellten Atemgases zugeführt wird und daß das vom Sauerstoffkonzentrator (7) erzeugte Atemgas durch eine Befülleinrichtung (1) hindurchgeleitet wird, in deren Bereich das Atemgas zur Befüllung der Druckgasflasche (5) von einem vom Sauerstoffkonzentrator (7) unabhängigen Verdichter (3) komprimiert wird sowie daß der Befüllvorgang der Druckgasflasche (5) in Abhängigkeit von mindestens einem Meßwert gesteuert wird, der in Abhängigkeit von einem aktuellen Atemgasbedarf im Bereich der Sauerstoffbrille (9) veränderlich ist.

2. Verfahren nach Anspruch 1, **dadurch gekennzeichnet, daß** eine mehrstufige Verdichtung des Atemgases durchgeführt wird.

3. Verfahren nach Anspruch 1 oder 2, **dadurch gekennzeichnet, daß** eine zweistufige Verdichtung des Atemgases durchgeführt wird.

4. Verfahren nach einem der Ansprüche 1 bis 3, **dadurch gekennzeichnet, daß** eine Messung der Sauerstoffkonzentration des Atemgases durchgeführt wird.

5. Verfahren nach einem der Ansprüche 1 bis 4, **dadurch gekennzeichnet, daß** eine Messung des Volumenflusses an Atemgas durchgeführt wird.

6. Verfahren nach einem der Ansprüche 1 bis 5, **dadurch gekennzeichnet, daß** ein Volumenfluß an Atemgas, der in Richtung auf die Sauerstoffbrille (9) geleitet wird, fest eingestellt wird und daß ein vom Sauerstoffkonzentrator (7) bereitgestellter Volumenfluß, der diesen eingestellten Wert übersteigt, dem Verdichter (3) zugeführt wird.

7. Verfahren nach einem der Ansprüche 1 bis 6, **dadurch gekennzeichnet, daß** von der Befülleinrichtung eine Verteilung von Atemgas durchgeführt wird, das von konstruktiv unterschiedlichen Sauerstoffkonzentratoren (7) bereitgestellt wird.

8. Verfahren nach einem der Ansprüche 1 bis 7, **dadurch gekennzeichnet, daß** eine Befeuchtung des in Richtung auf die Sauerstoffbrille (9) abgeleiteten Atemgases durchgeführt wird.

9. Verfahren nach einem der Ansprüche 1 bis 8, **dadurch gekennzeichnet, daß** im Bereich der Befülleinrichtung (1) mindestens ein Betriebsparameter angezeigt wird.

10. Vorrichtung zur Bereitstellung von Atemgas, die einen Sauerstoffkonzentrator sowie eine an den Sauerstoffkonzentrator anschließbare Sauerstoffbrille aufweist, **dadurch gekennzeichnet, daß** im Bereich einer Verbindungsleitung (24) zwischen dem Sauerstoffkonzentrator (7) und der Sauerstoffbrille (9) eine Befülleinrichtung (1) für Druckgasflaschen (5) angeordnet ist, daß die Befülleinrichtung (1) einen vom Sauerstoffkonzentrator (7) unabhängigen Verdichter (3) zur Kompression von Vorratsgas für die Druckgasflasche (5) sowie eine Steuereinrichtung (2) für eine Verteilung des vom Sauerstoffkonzentrator (7) erzeugten Atemgases aufweist und daß an die Steuereinrichtung (2) mindestens ein Sensor (11) zur Erfassung eines aktuellen Verbrauchs an Atemgas im Bereich der Sauerstoffbrille (9) angeschlossen ist.

11. Vorrichtung nach Anspruch 10, **dadurch gekennzeichnet, daß** der Verdichter (3) mehrstufig ausgebildet ist.

12. Vorrichtung nach Anspruch 10 oder 11, **dadurch gekennzeichnet, daß** der Verdichter (3) zweistufig ausgebildet ist.

13. Vorrichtung nach einem der Ansprüche 10 bis 12, **dadurch gekennzeichnet, daß** eine erste Stufe des Verdichters (3) im Bereich des Sauerstoffkonzentrators (7) und eine zweite Stufe des Verdichters (3) im Bereich der Befülleinrichtung (1) angeordnet ist.

14. Vorrichtung nach einem der Ansprüche 10 bis 13, **dadurch gekennzeichnet, daß** der Sensor (11) als ein Sensor zur Erfassung einer Sauerstoffkonzentration ausgebildet ist.

15. Vorrichtung nach einem der Ansprüche 10 bis 13, **dadurch gekennzeichnet, daß** der Sensor (11) als ein Sensor zur Erfassung eines Volumenflusses an Atemgas ausgebildet ist.

16. Vorrichtung nach einem der Ansprüche 10 bis 15, **dadurch gekennzeichnet, daß** die Befülleinrichtung (1) ein Einstellelement (12) zur Vorgabe eines Volumenflusses an Atemgas in Richtung auf die Sauerstoffbrille (9) aufweist.

17. Vorrichtung nach einem der Ansprüche 10 bis 16, **dadurch gekennzeichnet, daß** die Befülleinrichtung (1) einen Atemgaseingang (6) zum Anschluß relativ zueinander konstruktiv unterschiedlicher Sauerstoffkonzentratoren (7) aufweist.

18. Vorrichtung nach einem der Ansprüche 10 bis 16, **dadurch gekennzeichnet, daß** die Befülleinrichtung (1) einen Adapter (14) zum Anschluß relativ zueinander konstruktiv unterschiedlicher Sauerstoffkonzentratoren (7) aufweist.

19. Vorrichtung nach einem der Ansprüche 10 bis 18, **dadurch gekennzeichnet, daß** zwischen der Befülleinrichtung (11) und der Sauerstoffbrille (9) ein Befeuchter (10) angeordnet ist.

20. Vorrichtung nach einem der Ansprüche 10 bis 19, **dadurch gekennzeichnet, daß** die Befülleinrichtung (1) eine Anzeige (13) für mindestens einen Betriebsparameter aufweist.
